(19) **Europäisches Patentamt**
**European Patent Office**
**Office européen des brevets**

(11) **EP 2 011 429 B1**

(12) **EUROPEAN PATENT SPECIFICATION**

(45) Date of publication and mention
of the grant of the patent:
**09.11.2011 Bulletin 2011/45**

(51) Int Cl.:
***A61B 1/00*** *(2006.01)* ***G02B 23/24*** *(2006.01)*
***A61B 1/05*** *(2006.01)*

(21) Application number: **07742284.8**

(86) International application number:
**PCT/JP2007/058849**

(22) Date of filing: **24.04.2007**

(87) International publication number:
**WO 2007/125918 (08.11.2007 Gazette 2007/45)**

(54) **ENCAPSULATED ENDOSCOPE**

VERKAPSELTES ENDOSKOP

ENDOSCOPE ENCAPSULE

(84) Designated Contracting States:
**DE FR GB**

(30) Priority: **25.04.2006 JP 2006120789**

(43) Date of publication of application:
**07.01.2009 Bulletin 2009/02**

(73) Proprietors:
• **Olympus Medical Systems Corp.**
**Tokyo 151-0072 (JP)**
• **Olympus Corporation**
**Tokyo 151-0072 (JP)**

(72) Inventors:
• **SEGAWA, Hidetake**
**Tokyo 151-0072 (JP)**

• **ORIHARA, Tatsuya**
**Tokyo 151-0072 (JP)**
• **FUJIMORI, Noriyuki**
**Tokyo 151-0072 (JP)**

(74) Representative: **von Hellfeld, Axel**
**Wuesthoff & Wuesthoff**
**Patent- und Rechtsanwälte**
**Schweigerstrasse 2**
**81541 München (DE)**

(56) References cited:
**WO-A1-2005/070281     WO-A1-2006/040831**
**JP-A- 2003 260 023     JP-A- 2003 275 171**
**JP-A- 2006 068 488**

**Description**

TECHNICAL FIELD

This invention relates to a capsule endoscope introduced into a subject for sequentially picking up images inside the subject.

BACKGROUND ART

In recent years, a capsule endoscope of swallow type having the imaging function and the radio communication function has been proposed in the field of the endoscope. The capsule endoscope, after being swallowed from the mouth of the subject for observation (examination), moves inside the organs such as the stomach and the small intestine (i.e. inside the alimentary canal) while at the same time sequentially picking up images inside the alimentary canal of the subject at time intervals of, say, 0.5 second until it is naturally discharged from the subject.

The images picked up by the capsule endoscope, while being moved in the alimentary canal of the subject, are sequentially transmitted to an external receiving device from the capsule endoscope by radio communication. The receiving device has the function of communicating with the capsule endoscope and the memory function. The images sent by radio from the capsule endoscope in the subject are received by the receiving device and sequentially accumulated in the memory. The subject, carrying this receiving device, can behave freely from the time when the capsule endoscope is swallowed to the time when it is naturally discharged.

After the capsule endoscope introduced into the subject is naturally discharged from the subject, the doctor or the nurse causes an image display device to retrieve the images accumulated in the memory of the receiving device and to display the images (specifically, those in the alimentary canal) in the subject on the image display device. The doctor or the nurse, by observing the images of the interior of the subject displayed on the image display device, can diagnose the subject.

This capsule endoscope includes, in a capsule casing having a transparent dome at an end thereof, an imaging function including an imaging means for picking up images in the subject through the optical dome and an illumination means such as LED for illuminating the imaging field of the imaging means (see Patent Document 1, for example). This imaging means generally includes a solid image sensor such as CCD, a lens to focus the image of a subject on the light-receiving surface of the solid image sensor, and a cylindrical lens frame for holding the lens. In this case, the lens held by the lens frame converges the light reflected from the subject in the imaging field on the light-receiving surface of the solid image sensor. The solid image sensor, by photoelectric conversion of the light converged on the light-receiving surface by the lens, picks up an image in the subject corresponding to the image of the subject focused on the light-receiving surface.

Patent Document 1: International Publication No. 00/076391 Pamphlet

DISCLOSURE OF INVENTION

PROBLEM TO BE SOLVED BY THE INVENTION

The illumination means of the capsule endoscope emits the illumination light and illuminates the imaging field of the imaging means at the time when the imaging means described above picks up an image of the subject. By illuminating the imaging field in this manner, the imaging means can pick up an image in the subject as described above. In the conventional capsule endoscope described above, however, part of the illumination light emitted by the illumination means is reflected from the lens frame of the imaging means, and this reflected light, further reflected on the optical dome of the capsule casing, is liable to enter the lens of the imaging means. This poses the problem of a flare being generated.

Document JP 2006 068488 A concerns a capsule type endoscope comprising a lens frame provided in a hole of an illumination substrate, on which an illumination section is provided. A portion of the lens frame acts as a shielding unit.

Document JP 2003 275171 A concerns a capsule endoscope comprising a shielding member provided between an optical system and a plurality of illumination units.

This invention has been achieved in view of this situation, and the object thereof is to provide a capsule endoscope which can prevent the generation of a flare which otherwise might be caused by the light reflection on the lens frame.

MEANS FOR SOLVING PROBLEM

Some or all of the above problems are overcome by a capsule endoscope having the features of claim 1.

A capsule endoscope according to an aspect of the present invention has a solid image sensor introduced into a subject for picking up an image in the subject. The capsule endoscope includes a light-emitting unit for emitting illumination light for illuminating a view field of the solid image sensor; a lens frame for holding a lens to focus an image in the subject on a light-receiving surface of the solid image sensor, the lens frame having an upper end portion located at a position lower than an upper surface of the light-emitting unit; and a shield unit for shielding, from the illumination light, at least a partial area of the upper end portion of the lens frame which receives the illumination light.

In the capsule endoscope, the shield unit may have such a height that the shield unit crosses a plane which comes in contact with a crest of the upper surface of the light-emitting unit close to the lens frame and a crest of the upper end portion of the lens frame.

In the capsule endoscope, the crest of the upper end portion of the lens frame in contact with the plane may be located at the farthest end from the light-emitting unit.

The capsule endoscope includes a circuit board having a through hole into which the lens frame is inserted, the light-emitting unit being mounted on a surface of the circuit board, wherein the shield unit may be disposed between the upper end portion of the lens frame and the light-emitting unit on the circuit board.

In the capsule endoscope, the shield unit is formed integrally on the circuit board.

In the capsule endoscope, the shield unit may be formed by curing a paste-like resin coated on the circuit board.

In the capsule endoscope, the shield unit may be a chip part.

In the capsule endoscope, the shield unit may be formed to be such an endless shape as to surround the upper end portion of the lens frame.

In the capsule endoscope, the shield unit may be disposed directly on a side surface of the light-emitting unit facing at least the upper end portion of the lens frame.

In the capsule endoscope, the shield unit may be a frame member surrounding the side surface of the light-emitting unit.

EFFECT OF THE INVENTION

According to this invention, a sufficient internal space to accommodate the various parts on the circuit board can be secured in the capsule casing without increasing the size of the casing, while at the same time substantially preventing the light reflection at the upper end portion of the lens frame arranged on the circuit board. As a result, the invention exhibits the effect of promoting the size reduction of the endoscope on the one hand and realizing a capsule endoscope which can prevent the generation of a flare attributable to the light reflection on the lens frame on the other hand.

BRIEF DESCRIPTION OF DRAWINGS

FIG. 1 is a schematic side sectional view showing an example of the configuration of the capsule endoscope according to a first embodiment of the invention;

FIG. 2 is a schematic diagram illustrating the front surface of the capsule endoscope as viewed from the direction D1 shown in FIG. 1;

FIG. 3 is a schematic side sectional view showing an example of a shield unit externally mounted between the upper end portion of the lens frame and the corresponding light-emitting unit on the front surface of the illumination board;

FIG. 4 is a schematic side sectional view explaining the shield operation of the shield units of the capsule endoscope according to the first embodiment;

FIG. 5 is a schematic front view showing an example of the configuration of the capsule endoscope according to a first modification of the first embodiment of the invention;

FIG. 6 is a schematic front view showing an example of the configuration of the capsule endoscope according to a second modification of the first embodiment of the invention;

FIG. 7 is a schematic side sectional view showing an example of the configuration of the capsule endoscope according a third modification of the first embodiment of the invention;

FIG. 8 is a schematic side sectional view showing an example of the configuration of the capsule endoscope according to a second embodiment of the invention;

FIG. 9 is a schematic diagram illustrating the front surface of the capsule endoscope as viewed from the direction D1 shown in FIG. 8;

FIG. 10 is a schematic side sectional view showing an example of the shield units formed integrally on the illumination board;

FIG. 11 is a schematic front view showing an example of the configuration of the capsule endoscope according to a modification of the second embodiment of the invention;

FIG. 12 is a schematic side sectional view showing an example of the shield units of the capsule endoscope according to a modification of the second embodiment of the invention;

FIG. 13 is a schematic side sectional view showing an example of the configuration of the capsule endoscope according to a third embodiment of the invention;

FIG. 14 is a schematic diagram illustrating the front surface of the capsule endoscope as viewed from the direction D1 shown in FIG. 13;

FIG. 15 is a schematic side sectional view showing an example of the shield units arranged directly on the side surface of the light-emitting unit;

FIG. 16 is a schematic side sectional view showing an example of the configuration of the capsule endoscope according to a fourth embodiment of the invention;

FIG. 17 is a schematic diagram illustrating the front surface of the capsule endoscope as viewed from the direction D1 shown in FIG. 16;

FIG. 18 is a schematic side sectional view showing an example of the upper end portion of the lens frame reflecting the illumination light from the light-emitting unit to a position outside the view field of the solid image sensor;

FIG. 19 is a schematic sectional view explaining the structure of the upper end portion of the lens frame in more detail;

FIG. 20 is an enlarged schematic diagram showing the lens frame 44e in FIG. 19; and

FIG. 21 is a schematic side sectional view explaining the reflect operation of the upper end surface of the lens frame reflecting the illumination light from the light-emitting units to a position outside the view field of the solid image sensor.

EXPLANATIONS OF LETTERS OF NUMERALS

| 1, 1a, 1b, 1c, 21, 21a, 31, 41 | Capsule endoscope |
| 2 | Casing |
| 2a | Case body |
| 2b | Optical dome |
| 3, 23, 33 | Illumination system |
| 3a, 33a | Light-emitting unit |
| 3b, 23b, 24 | Illumination board |
| 3c | Function part |
| 4, 44 | Imaging system |
| 4a | Solid image sensor |
| 4b, 44b | Optical system |
| 4c | Imaging board |
| 4d | Lens |
| 4e, 44e | Lens frame |
| 5 | Radio communication system |
| 5a | Wireless board |
| 5b | Antenna |
| 5c | Power supply board |
| 6 | Power supply system |
| 6a | Battery |
| 6b | Power supply board |
| 6c | On-off switch |
| 7 | Control unit |
| 8, 15, 16, 17, 26, 28, 32 | Shield unit |
| 27, 29 | Cavity portion |
| 45 | Upper end surface |

BEST MODE FOR CARRYING OUT THE INVENTION

preferred embodiments of a capsule endoscope according to the invention is explained in detail below with reference to the drawings. This invention is not limited by these embodiments.

First embodiment

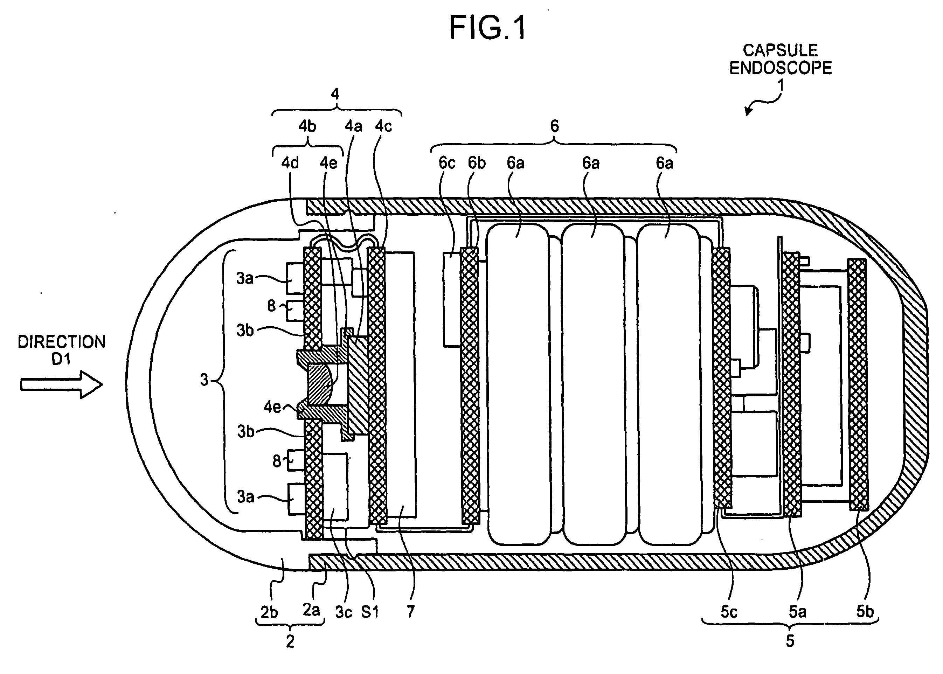

FIG. 1 is a schematic side sectional view showing an example of the configuration of the capsule endoscope according to a first embodiment of the invention. The description that follows represents an example of the capsule endoscope introduced from the mouth or the like of a person constituting a subject to sequentially pick up images of interior (specifically, the interior of the alimentary canal) of the subject.

As shown in FIG. 1, the capsule endoscope 1 according to the first embodiment includes a casing 2 formed in the shape of capsule, an illumination system 3 for illuminating the interior of the subject, an imaging system 4 for picking up images of the interior of the subject illuminated by the illumination system 3, and a radio communication system 5 for transmitting, outside by radio, the images of the interior of the subject sequentially picked up by the imaging system 4. The capsule endoscope 1 also includes a power supply system 6 for supplying the drive power to each component part, a control unit 7 for controlling the drive of each component part and shield units 8 for blocking the illumination light to the upper end portion of the lens frame of the imaging system 4.

The casing 2 is of capsule type formed to such a size as to be easily introduced into the subject. Specifically, the casing 2 is realized by a case body 2a formed in the shape of a capsule, and an optical dome 2b mounted on the front end portion of the case body 2a. The case body 2a is a cylindrical case with the front end thereof open and the rear end thereof closed like a dome, and accommodates therein the illumination system 3, the imaging system 4, the radio communication system 5, the power supply system 6, the control unit 7 and the shield units 8. The optical dome 2b is a substantially transparent dome-like member high in light transmittance and mounted at the front end of the case body 2a to close the front end (open end). The casing 2 formed of the case body 2a and the optical dome 2b accommodates, in liquidtight fashion, the component parts (for example, the illumination system 3, the imaging system 4, the radio communication system 5, the power supply system 6, the control unit 7 and the shield units 8) of the capsule endoscope 1.

The illumination system 3 functions as an illumination means for illuminating the interior of the subject imaged by the imaging system 4. Specifically, the illumination system 3 includes the light-emitting units 3a for emitting the light to illuminate the interior of the subject through the optical dome 2b, the illumination board 3b formed with a circuit to realize the function of the illumination system 3 and a function part 3c having a chip part such as a chip resistor or a chip capacitor.

The light-emitting units 3a are each a luminescent element such as LED and emit the illumination light (white light, for example) to illuminate the view field of the solid image sensor (described later) of the imaging system 4. The illumination board 3b, being a rigid circuit board formed in disk, for example, has the plural light-emitting units 3a mounted in the neighborhood of the outer periphery of the front surface (the surface close to the optical dome 2b shown in FIG. 1) and the function part 3c mounted at a predetermined position on the rear surface thereof. The plural light-emitting units 3a mounted on the illumination board 3b illuminate the interior of the subject (i.e. the view field of the solid image sensor of the imaging system 4) over the optical dome 2b by emitting the illumination light. A through hole in which the lens frame (described later) of the imaging system 4 is inserted is formed at the central part of the illumination board 3b.

The imaging system 4 functions as an imaging means for picking up images in the subject. Specifically, the imaging system 4 includes a solid image sensor 4a such as CCD or CMOS for picking up images in the subject, an optical system 4b to focus the images in the subject on the light-receiving surface of the solid image sensor 4a and an imaging board 4c formed with a circuit for realizing the function of the imaging system 4.

The solid image sensor 4a images a subject in the view field illuminated by the light-emitting units 3a. Specifically, the solid image sensor 4a has a light-receiving surface for receiving the light from the subject located within the view field, and picks up the images of the subject (i.e. the images in the subject) by photoelectric conversion of the light received from the subject through this light-receiving surface.

The optical system 4b includes a lens 4d to focus the image in the subject on the light-receiving surface of the solid image sensor 4a and a lens frame 4e for holding the lens 4d. The lens 4d has a view angle specifying the view field of the solid image sensor 4a, and converging the light from the subject on the light-receiving surface of the solid image sensor 4a, focuses the image of the subject (i.e. the image in the subject located in the view field) on the light-receiving surface of the solid image sensor 4a. The lens frame 4e has a cylindrical structure with open ends and holds the lens 4d in the cylinder. Specifically, the lens frame 4e holds the lens 4d in the cylinder in the neighborhood of the opening at an end (i.e. the upper end portion) on the one hand and is fixed on the solid image sensor 4a with the opening at the other end in registry with the light-receiving surface. Also, the neighborhood of the upper end portion of the lens frame 4e is inserted in the through hole formed at the central part of the illumination board 3b described above. In this case, the upper end portion of the lens frame 4e is exposed to the front side of the illumination board 3b and arranged facing the optical dome 2b at a lower position than the upper surface of the light-emitting unit 3a. In this way, the lens frame 4e inserted in the through hole of the illumination board 3b holds the lens 4d at a position lower than the upper surface of the light-emitting units 3a mounted on the front surface of the illumination board 3b.

The imaging board 4c is a rigid circuit board formed in the shape of a disk, for example, and, electrically connected with the illumination board 3b by a flexible board or the like. The solid image sensor 4a is mounted on the surface of the imaging board 4c facing the illumination board 3b as shown in FIG. 1. In this case, an internal space S1 for arranging a function part 3c mounted on the illumination board 3b is formed between the imaging board 4c and the illumination board 3b facing each other. In this case, the lens frame 4e is inserted in the through hole of the illumination board 3b in such a manner that the upper end portion of the lens frame 4e is arranged at a position lower than the upper surface of the light-emitting units 3a mounted on the front surface of the illumination board 3b. Thus, the internal space S1 is sufficiently large to arrange therein the function part 3c mounted on the illumination board 3b.

The radio communication system 5 functions as a radio communication means for sequentially transmitting, by radio, the internal images of the subject picked up by the imaging system 4 to an external receiving device (not shown). Specifically, the radio communication system 5 includes a wireless board 5a having a radio unit thereon, an antenna 5b for transmitting the radio signal containing the images in the subject to outside and a power supply board 5c having a DC/DC converter or the like. The wireless board 5a and the power supply board 5c are electrically connected to each other by, for example, a flexible board.

The wireless board 5a is a rigid discal board formed with a circuit for realizing the function of the radio communication system 5, and has a radio unit mounted thereon. The radio unit of the wireless board 5a receives the image signal containing the internal images of the subject picked up by the solid image sensor 4a, and generates a radio signal containing the internal images of the subject by modulating the received image signal. The antenna 5b sequentially transmits the radio signals generated by the radio unit to the external receiving device (not shown). The power supply board 5c supplies the radio unit of the wireless board 5a with the drive power supplied from the power supply system 6.

The power supply system 6 supplies the drive power to each of the component parts (for example, the illumination system 3, the imaging system 4, the radio communication system 5 and the control unit 7) of the capsule endoscope 1. Specifically, the power supply system 6 includes a battery 6e having a predetermined power, a power supply board 6b formed with a circuit for realizing the function of the power supply system 6 and an on-off switch 6c for supplying the drive power.

The battery 6a is button-type dry cells such as the silver oxide cells, and as shown in FIG. 1, as many cells 6a (three, for example) as required are connected between the power supply boards 5c and 6b. The battery 6a supplies the drive power to each component part of the capsule endoscope 1 through the power supply boards 6b, 5c and the like. The on-off switch 6c is a reed switch for switching the on-off operation by, for example, an external magnetic force and arranged on the power supply board 6b. The on-off switch 6c, through this on-off switching operation, controls the drive power supplied to the component parts of the capsule endoscope 1 from the battery 6a. The power supply board 6b is a rigid circuit board formed in the shape of a disk, for example, and electrically connected to the imaging board 4c and the power supply board 5c described above by a flexible board. The number of the cells 6a connected between the power supply boards 5c and 6b is not specifically limited to three but may be any number that can supply the required drive power to each component part of the capsule endoscope 1.

The control unit 7 is mounted on the imaging board 4c described above, for example, and controls the drive operation of each component part of the capsule endoscope 1. Specifically, the control unit 7 controls the each drive operation of the light-emitting units 3a of the illumination system 3 described above, the solid image sensor 4a of the imaging system 4 and the radio unit arranged on the wireless board 5a of the radio communication system 5. This control unit 7 performs the various signal processing operations such as to generate the image data in collaboration with the solid image sensor 4a on the one hand, and exhibits the function as a timing generator permitting the illumination system 3 and the imaging system 4 to sequentially pick up the images in the subject at predetermined time intervals on the other hand. Further, the control unit 7 stores various parameters for the lines and frames of the images.

The shield units 8 function as a shield means for blocking the illumination light which otherwise might be radiated on the upper end portion of the lens frame 4e inserted in the through hole of the illumination board 3b. Specifically, each shield unit 8 is externally mounted using the solder or adhesive between the corresponding one of the light-emitting units 3a and the upper end portion of the lens frame 4e on the front surface of the illumination board 3b. In the shield units 8, part of the light emitted from the light-emitting units 3a and propagated toward the upper end portion of the lens frame 4e from the light-emitting units 3a (i.e. the illumination light which would be received by the upper end portion of the lens frame 4e from the light-emitting units 3a in the absence of the shield units 8) is shielded from the upper end portion of the lens frame 4e. By shielding part of the illumination light from the upper end portion of the lens frame 4e in this manner, the shield units 8 prevent the illumination light from being reflected on the upper end portion of the lens frame 4e. As a result, the shield units 8 suppress the generation of a flare which otherwise might be caused by the light reflection on the lens frame 4e.

In the case where the plural light-emitting units 3a are mounted on the front surface of the illumination board 3b, each shield unit 8 is arranged in the space between the corresponding one of the plural light-emitting units 3a and the upper end portion of the lens frame 4e on the front surface of the illumination board 3b. Also, the shield units 8 are desirably formed of a material such as a metal or a resin easily erected on the front surface of the illumination board 3b.

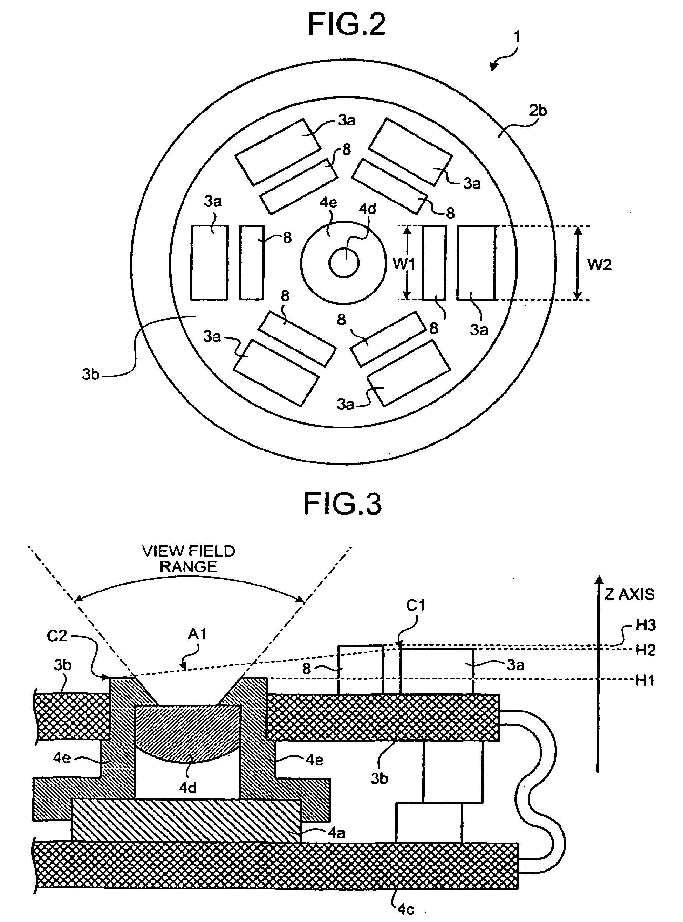

Next, the shield units 8 for shielding the illumination light of the light-emitting units 3a from the upper end portion of the lens frame 4e inserted in the through hole of the illumination board 3b are explained in detail. FIG. 2 is a schematic diagram illustrating the front surface of the capsule endoscope 1 as viewed from the direction D1 in FIG. 1. FIG. 3 is a schematic side sectional view showing an example of the shield unit 6 mounted externally between the upper end portion of the lens frame 4e and the corresponding light-emitting unit 3a on the front surface of the illumination board 3b.

As shown in FIG. 2, the upper end portion of the lens frame 4e holding the lens 4d is exposed to the front surface of the illumination board 3b from the through hole at the central part of the illumination board 3b. Also, six light-emitting units 3a, for example, are mounted at rotationally symmetric positions around the optical axis of the lens 4d in the neighborhood of the outer periphery of the front surface of the illumination board 3b. In this case, the each shield unit 8 is arranged in each space between the six light-emitting units 3a and the lens frame 4e on the front surface of the illumination board 3b. The number of the light-emitting units 3 mounted on the front surface of the illumination board 3b is not specifically limited to six, but any number will do which can illuminate the view field of the solid image sensor 4a sufficiently to vividly pick up the internal images of the subject.

The shield units 8 interposed between the light-emitting units 3a and the upper end portion of the lens frame 4e each have a width W1 equal to or larger than the width W2 of the light-emitting unit 3a. In this case, each shield unit 8 is arranged in such a manner that the corresponding one of the light-emitting units 3a is located in the range between the two parallel planes including the side end portions, respectively, defining the width W1 (i..e. the two parallel planes distant by W1 from each other).

Also, the upper end portion of the lens frame 4e inserted in the through hole of the illumination board 3b, as described above, is located at a position lower than the upper surface of the light-emitting units 3a mounted on the front surface of the illumination board 3b. Specifically, as shown in FIG. 3, assuming that the Z axis is defined along the height parallel to the optical axis of the lens 4d and directed outward of the capsule endoscope through the optical dome 2b, the height H1 of the upper end portion of the lens frame 4e is lower than the height H2 of the upper surface of the light-emitting units 3a mounted on the front surface of the illumination board 3b. Each shield unit 8 interposed between the upper end portion of the lens frame 4e and the corresponding light-emitting unit 3a has a sufficient height H3 to cross the plane A1 in contact with the crest C1 between the upper surface and the side surface of the light-emitting unit 3a and the crest C2 of the upper end portion of the lens frame 4e. In this case, the shield unit 8 having this height H3 is located outside the view field defined by the lens 4d without obstructing the view field of the solid image sensor 4a.

The heights H1, H2, H3 of the respective parts are defined by the Z axis from a predetermined reference plane shown in FIG. 3. Of these heights, the height H3 equals the height of the upper surface of the shield units 8. The crest C2 of the upper end portion of the lens 4e in contact with the plane A1 described above is located at a position farthest from the light-emitting units 3a.

These shield units 8 can completely hide the light-emitting units 3a from the upper end portion of the lens frame 4e, with the result that the light propagating from the light-emitting units 3a toward the upper end portion of the lens frame 4e (i.e. part of the illumination light described above) can be positively shielded from the lens frame 4e. This shield operation of the shield units 8 prevents the light reflection at the upper end portion of the lens frame 4e, thereby preventing the occurrence of a flare which otherwise might be caused by the light reflection on the lens frame 4e.

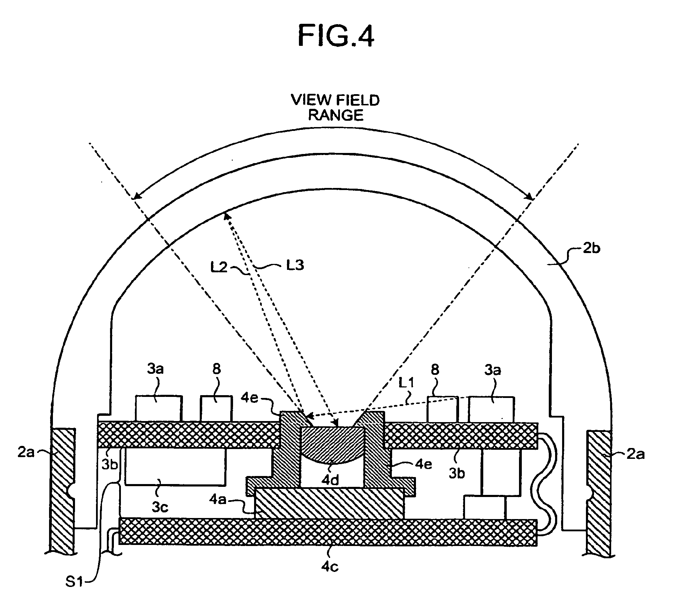

Next, an explanation is given about the shield operation in which the upper end portion of the lens frame 4e is shielded by the shield units 8 mounted externally between the light-emitting units 3a and the upper end portion of the lens frame 4e. FIG. 4 is a schematic side sectional view explaining the shield operation of one of the shield units 8 of the capsule endoscope 1 according to the first embodiment. As described above, the light propagated from the light-emitting unit 3a toward the upper end portion of the lens frame 4e is positively blocked by the shield unit 8. In this case, the shield unit 8, as shown in FIG. 4, completely blocks the path of the light (the light path L1, for example) directed from the corresponding light-emitting unit 3a toward the upper end portion of the lens frame 4e.

The internal space S1 described above can be secured effectively without increasing the size of the capsule endoscope (the size of the casing thereof) in such a manner that the side surface of the cylindrical portion of the lens frame 4e as close to the upper end portion thereof as possible is fitted in the through hole of the illumination board 3b thereby to separate the illumination board 3b and the imaging board 4c as far as possible from each other. As a result, a sufficient space to arrange the function part 3c of the illumination board 3b can be secured without increasing the body size of the capsule endoscope.

The upper end portion of the lens frame 4e fitted in the through hole of the illumination board 3b as described above, however, is located at a position lower than the upper surface of the light-emitting units 3a on the one hand and the lens 4d is held at a position lower than the upper surface of the light-emitting units 3a on the other hand. In the case where the light-emitting units 3a and the lens frame 4e are arranged in these relative positions in the absence of the shield units 8, part of the illumination light emitted from the light-emitting units 3a would propagate along the light path L1, for example, and reaching the upper end portion of the lens frame 4e, would be reflected at the upper end portion of the lens frame 4e. The light reflected at the upper end portion of the lens frame 4e would propagate along the light path L2, for example, reaching a position on the optical dome 2b at a position in the range of the view field thereof (within the range of the view field of the solid image sensor 4a) and would be reflected by the optical dome 2b at the position within the particular view field. After that, the light reflected at the position within the range of the view field of the optical dome 2b would propagate along the light path L3, for example, and enter the lens 4d. As a result, a flare would be generated by the light reflection at the upper end portion of the lens frame 4e.

In the capsule endoscope 1 according to the first embodiment of the invention having the shield units 8 between the light-emitting units 3a and the upper end portion of the lens frame 4e as described above, in contrast, the shield units 8 can block all the light paths such as the light path L1 from the light-emitting units 3a toward the upper end portion of the lens frame 4e, so that the lens frame 4e can be positively shielded from the light rays propagating from the light-emitting units 3a toward the upper end portion of the lens frame 4e. As a result, the shield units 8 can prevent the light reflection at the upper end portion of the lens frame 4e while at the same time preventing the generation of a flare which otherwise might be caused by the light reflection at the upper end portion of the lens frame 4e.

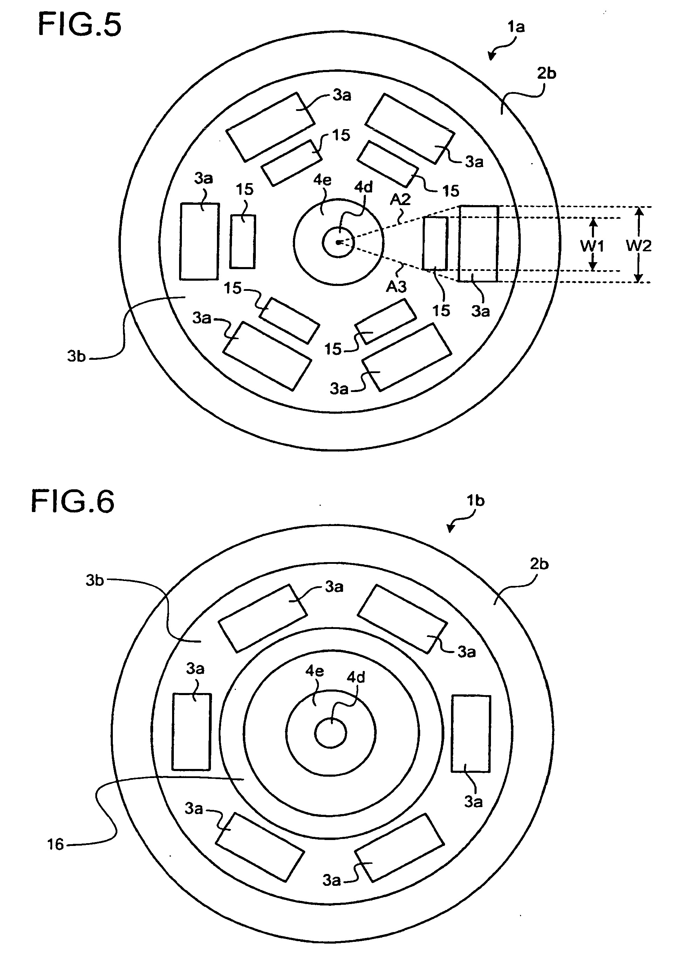

Next, the capsule endoscope according to a first modification of the first embodiment of the invention is explained. FIG. 5 is a schematic front view showing an example of the configuration of the capsule endoscope according the first modification of the first embodiment of the invention. As described above, the shield units externally mounted between the light-emitting units 3a and the upper end portion of the lens frame 4e on the front surface of the illumination board 3b may not necessarily be exclusive shield members (for example, the shield units 8 described above) for shielding the upper end portion of the lens frame 4e form the illumination light but may alternatively be chip parts such as chip resistors or chip capacitors mounted on the illumination board 3b. Specifically, as shown in FIG. 5, the capsule endoscope 1a according to the first modification of the first embodiment has shield units 15 in place of the shield units 8 of the capsule endoscope 1 according to the first embodiment described above. The other parts of the configuration are identical with and designated by the same reference numerals, respectively, as the corresponding ones of the first embodiment.

The shield units 15 are, for example, multipurpose chip parts such as chip resistors or chip capacitors mounted on the illumination board 3b. The shield units 15 constituting such chip parts are mounted using solder or the like between the upper end portion of the lens frame 4e and the light-emitting units 3a on the front surface of the illumination board 3b. In this case, the shield units 15 as the chip parts are electrically connected to the illumination board 3b.

Also, the shield units 15, substantially similarly to the shield units 8 described above, each have a sufficient height H3 to cross the plane A1 (see FIG. 3) in contact with the crest C1 of the light-emitting units 3a and the crest C2 of the upper end portion of the lens frame 4e. The width W1 of each shield unit 15, on the other hand, is sometimes smaller than the width W2 of the corresponding light-emitting unit 3a. In such a case, the shield units 15, as shown in FIG. 5, are arranged in such a manner as to cross or contact both the planes A2 and A3 passing through the crest of the light-emitting units 3a and the optical axis of the lens 4d. The shield units 15 arranged in such a position can shield at least the partial area of the upper end portion of the lens frame 4e which receives the illumination light from the light-emitting units 3a. The partial area of the lens frame 4e as defined here is the one which would receive the illumination light from the light-emitting units 3a in the absence of the shield units 15, such as a slope or a crest facing the light-emitting units 3a, for example.

The plane A2 is the one passing through one of the two crests located close to the lens frame 4e between the side surfaces of each light-emitting unit 3a and the optical axis of the lens 4d, and the plane A3 is the one passing through the other of the two crests and the optical axis of the lens 4d.

In the case where this partial area of the lens frame 4e receives the illumination light from the light-emitting units 3a, the flare described above would be generated by the light reflected on the particular partial area of the lens frame 4e. The shield units 15 described above, on the other hand, can block all the light paths from the light-emitting units 3a toward the partial area of the lens frame 4e and thus can positively shut off the light rays propagating from the light-emitting units 3a toward such a partial area of the lens frame 4e. As a result, the shield units 15 enjoy substantially the same operation-effect as the shield units 8 of the first embodiment described above, and can suppress the flare which would otherwise be generated by the reflection of the light on the upper end portion of the lens frame 4e.

Also, the shield units 15 are realized using the multipurpose chip parts described above, and therefore, so low in unit cost as to be capable of being mounted together with the other parts (for example, the light-emitting units 3a and the function part 3c) on the illumination board 3b. As a result, as compared with the conventional capsule endoscope having no shield function, the capsule endoscope 1a having the shield units 15 (i.e. having the shield function described above) can be realized without substantially increasing the production cost.

Next, the capsule endoscope according to a second modification of the first embodiment of the invention is explained. FIG. 6 is a schematic front view showing an example of the configuration of the capsule endoscope according to the second modification of the first embodiment of the invention. As described above, shield units externally mounted between the plural light-emitting units 3a and the upper end portion of the lens frame 4e on the front surface of the illumination board 3b is not limited to the plural shield units externally mounted on the front surface of the illumination board 3b corresponding to each plural light-emitting units 3a (for example, the plural shield units 8). Instead, an endless shield unit may alternatively be mounted externally in such a manner as to surround the upper end portion of the lens frame 4e. Specifically, as shown in FIG. 6, the capsule endoscope 1b according to the second modification of the first embodiment has an annular shield unit 16 in place of the plural shield units 8 of the capsule endoscope 1 according to the first embodiment. The other parts of the configuration are identical with the corresponding ones, respectively, of the first embodiment and designated by the same reference numerals, respectively.

The shield unit 16 is, for example, a shield member formed in a ring and externally mounted in such a manner as to surround the lens frame 4e at a position between the upper end portion of the lens frame 4e and the light-emitting units 3a on the front surface of the illumination board 3b. In this case, the shield unit 16 is externally bonded to the front surface of the illumination board 3b using, for example, the solder or an adhesive. Also, the shield unit 16, substantially similarly to the plural shield units 8 described above, has a sufficient height H3 to cross the plane A1 (see FIG. 3) in contact with the crest C1 of each light-emitting unit 3a and the crest C2 of the upper end portion of the lens frame 4e.

This annular shield unit 16, like the plural shield units 8 described above, can block all the light paths (for example, the light path L1 described above) directed from the plural light-emitting units 3a toward the upper end portion of the lens frame 4e on the illumination board 3b. In this way, the lens frame 4e can be positively shielded from all the light rays propagating from the plural light-emitting units 3a toward the upper end portion of the lens frame 4e. As a result, the single shield unit 16, like the plural shield units 8 described above, can prevent the light reflection at the upper end portion of the lens frame 4e and thereby suppresses the generation of a flare which otherwise might be caused by the light reflection at the upper end portion of the lens frame 4e.

Also, in the capsule endoscope 1b having this annular shield unit 16, unlike in the capsule endoscope 1 according to the first embodiment described above, the plural shield units 8 corresponding to the plural light-emitting units 3a are not required, but the single annular shield unit 16 may be interposed between the light-emitting units 3a and the upper end portion of the lens frame 4e. Thus, the number of the shield units externally mounted on the front surface of the illumination board 3b can be reduced to one, and the labor of externally mounting the shield units can be saved while at the same time reducing the production cost of the capsule endoscope having this shield unit.

The shield unit 16 externally mounted between the light-emitting units 3a and the upper end portion of the lens frame 4e is not necessarily in the form of ring but may be an endless one in any shape surrounding the upper end portion of the lens frame 4e. Specifically, the endless shield unit 16, as shown in FIG. 6, may be formed in either a ring (circle, ellipse) or a polygon. Also, the endless shield unit 16 is desirably formed of a member of such a material as a metal or a resin easily erected on the front surface of the illumination board 3b.

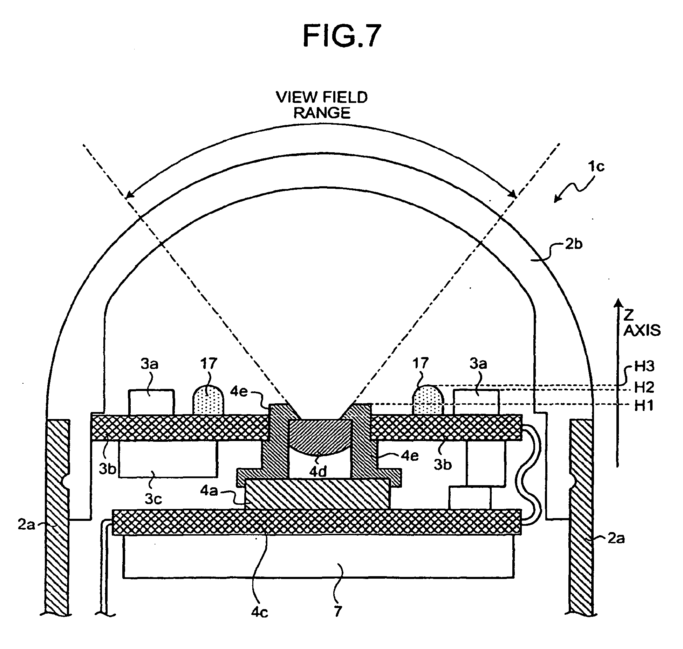

Next, a third modification of the first embodiment of the invention is explained. FIG. 7 is a schematic side sectional view showing an example of the configuration of the capsule endoscope according to a third modification of the first embodiment of the invention. The shield units for shielding the illumination light from the light-emitting units 3a toward the upper end portion of the lens frame 4e are not limited to those described above, but alternatively so configured that the paste-like resin is coated and cured at positions between the light-emitting units 3a and the upper end portion of the lens frame 4e on the front surface of the illumination board 3b.

Specifically, as shown in FIG. 7, the capsule endoscope 1c according to the third modification of the first embodiment has shield units 17 in place of the shield units 8 of the capsule endoscope 1 according to the first embodiment described above. The other parts of the configuration are identical with the corresponding ones of the first embodiment and designated by the same reference numerals, respectively.

The shield units 17 are formed on the illumination board 36 by curing the paste-like resin such as the sealing agent used for semiconductor fabrication, for example. Specifically, the shield units 17 are formed in such a manner that the paste-like resin is coated using the dispenser at positions between the plural light-emitting units 3a and the upper end portion of the lens frame 4e on the front surface of the illumination board 3b, and the paste-like resin coated at such positions is cured by heat treatment, UV radiation or the like. In this case, the paste-like resin may be coated at plural positions on the illumination board 3b to block the paths between the plural light-emitting units 3a and the upper end portion of the lens frame 4e, or formed as a single unit in endless fashion (in a ring, for example) surrounding the upper end portion of the lens frame 4e. Specifically, either the plural shield units 17 having the width W1 corresponding to the plural light-emitting units 3a may be formed by curing the paste-like resin, or a single endless one like the shield unit 16 according to the second modification of the first embodiment described above may surround the upper end portion of the lens frame 4e.

The shield unit(s) 17 formed by curing the paste-like resin, substantially similarly to the shield units 8 or the endless shield unit 16 described above, has a sufficient height H3 to cross the plane A1 (see FIG. 3) in contact with the crest C1 of each light-emitting unit 3a and the crest C2 of the upper end portion of the lens frame 4e.

This shield unit 17, like the plural shield units 8 or the endless shield unit 16 described above, can block all the light paths (for example, the light path L1 described above) directed from the plural light-emitting units 3a toward the upper end portion of the lens frame 4e on the illumination board 3b. In this way, the lens frame 4e can be positively shielded from all the light rays propagating from the plural light-emitting units 3a toward the upper end portion of the lens frame 4e. As a result, the shield unit 17, like the plural shield units 8 or the endless shield unit 16 described above, can prevent the light reflection at the upper end portion of the lens frame 4e while at the same time suppressing the generation of a flare which otherwise might be caused by the light reflection at the upper end portion of the lens frame 4e.

The shield unit 17 like this is formed by curing the paste-like resin coated on the illumination board 3b using the dispenser as described above. By controlling the amount of the paste-like resin coated by the dispenser or the track of the nozzle, therefore, the shape, height and the width of the shield unit 17 can be easily controlled as desired.

As explained above, according to the first embodiment and the first to third modifications of the first embodiment of the invention, the shield unit is interposed between the light-emitting units for emitting the illumination light to illuminate the view field of the solid image sensor and the upper end portion of the lens frame holding the lens to focus the image of the subject on the light-receiving surface of the solid image sensor.

This upper end portion of the lens frame is located lower than the upper surface of the light-emitting units, and the shield unit is so configured that at least that partial area (for example, the upper end surface or the crest facing the light-emitting units) of the upper end portion of the lens frame which receives the illumination light from the light-emitting units are shielded from the illumination light of the light-emitting units. In this way, a sufficient internal space can be secured in the casing to accommodate various parts on the circuit board without increasing the size of the capsule casing while at the same time preventing the light reflection on the upper end portion of the lens frame (especially, the partial area such as the upper end surface or the crest facing the light-emitting units). As a result, the capsule endoscope is realized in which the size reduction of the apparatus is promoted on the one hand and the flare which otherwise might be caused by the light reflection on the upper end portion of the lens frame 4e can be suppressed on the other hand.

Also, according to the first modification of the first embodiment of the invention, multipurpose chip parts such as chip resistors or chip capacitors are mounted between the light-emitting units and the upper end portion of the lens frame as the shield units described above. Therefore, the unit cost of the parts forming the shield units can be reduced on the one hand, and the shield unit can be mounted on the circuit board together with the other parts (such as the light-emitting units) on the other hand. As a result, the production cost of the capsule endoscope having this shield unit can be reduced.

Further, according to the second modification of the first embodiment of the invention, the endless shield unit is arranged in such a manner as to surround the lens frame between the light-emitting units and the upper end portion of the lens frame, and therefore, the number of the shield units arranged can be reduced to one regardless of the number of the light-emitting units. As a result, the labor for externally mounting the shield units can be saved and the production cost of the capsule endoscope having this shield unit can be reduced.

Further, according to the third modification of the first embodiment of the invention, the shield unit is formed by curing the paste-like resin coated between the light-emitting units and the upper end portion of the lens frame. By controlling the amount of the paste-like resin coated by the dispenser or the track of the nozzle, therefore, the position, shape, height and the width of the shield unit can be easily controlled as desired.

Second embodiment

Next, a second embodiment of the invention is explained. According to the first embodiment and the first to third modifications of the first embodiment of the invention, the shield units (for example, the shield units 8, 15, 16, 17) independent of the illumination board 3b are arranged at a position between the light-emitting units 3a and the upper end portion of the lens frame 4e on the front surface of the illumination board 3b. According to the second embodiment, on the other hand, a shield unit for shielding the upper end portion of the lens frame 4e from the illumination light of the light-emitting units 3a is formed integrally with the illumination board.

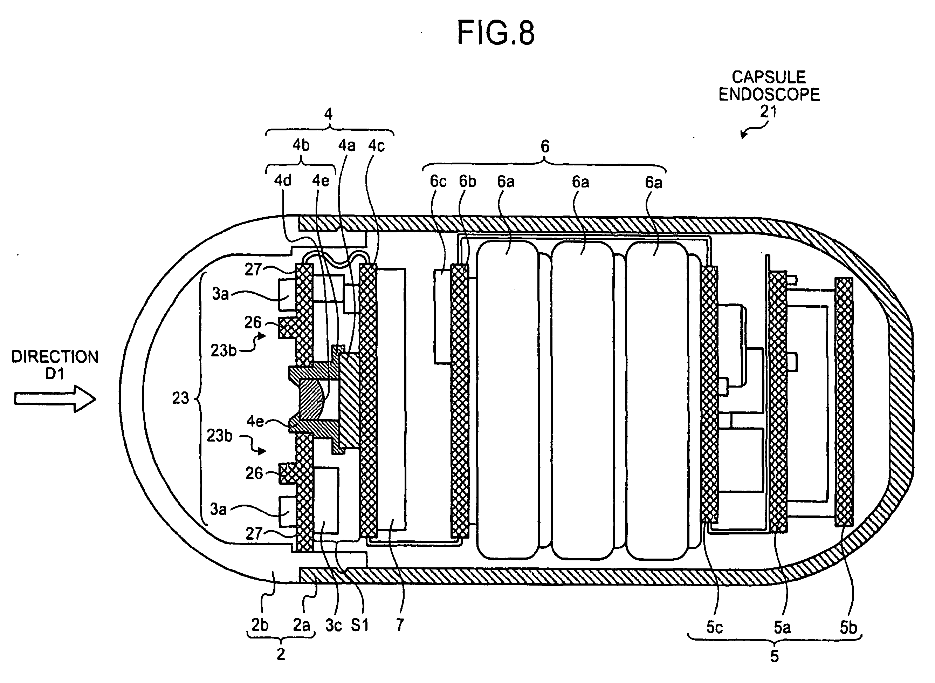

FIG. 8 is a schematic side sectional view showing an example of the configuration of the capsule endoscope according to the second embodiment of the invention. As shown in FIG. 8, the capsule endoscope 21 according to the second embodiment has an illumination system 23 in place of the illumination system 3 of the capsule endoscope 1 according to the first embodiment described above. This illumination system 23 has an illumination board 23b in place of the illumination board 3b of the illumination system 3 of the capsule endoscope 1 described above. In this illumination board 23b, the shield unit 26 is formed integrally in place of the shield units 8 of the capsule endoscope 1 described above at a position between the light-emitting units 3a and the upper end portion of the lens frame 4e. The other parts of the configuration are identical with the corresponding ones, respectively, of the first embodiment and designated by the same reference numerals, respectively.

The illumination board 23b is a disk-like rigid board formed with the circuit for realizing the function of the illumination system 23, and almost like in the capsule endoscope 1 according to the first embodiment described above, includes the plural light-emitting units 3a and the upper end portion of the lens frame 4e. In this illumination board 23b, the shield unit 26 is formed integrally between the light-emitting units 3a and the upper end portion of the lens frame 4e, and a cavity portion 27 for mounting the plural light-emitting portions 3a is formed on the outer periphery of the shield unit 26.

The shield unit 26 is a protrusion formed integrally on the illumination board 23b and functions as a shield means in which the upper end portion of the lens frame 4e inserted in the through hole of the illumination board 23b is shielded from the illumination light of the light-emitting units 3a. Specifically, the shield unit 26 is formed by protruding the front surface portion of the illumination board 23b located between the light-emitting units 3a and the upper end portion of the lens frame 4e. In this case, the shield unit 26 is formed in endless fashion (for example, as a ring) in such a manner as to surround the upper end portion of the lens frame 4e inserted in the through hole of the illumination board 23b. This shield unit 26, like the annular shield unit 16 described above, positively blocks all the light rays propagating from the plural light-emitting units 3a toward the upper end portion of the lens frame 4e.

The cavity portion 27 is located at a lower level than the shield unit 26 on the front surface of the illumination board 23b and used for mounting the plural light-emitting units 3a. Specifically, the cavity portion 27 is formed on the outer periphery of the shield unit 26 by forming the particular shield unit 26 (protruded portion) on the front surface of the illumination board 23b. This cavity portion 27 has mounted thereon, for example, the plural light-emitting units 3a.

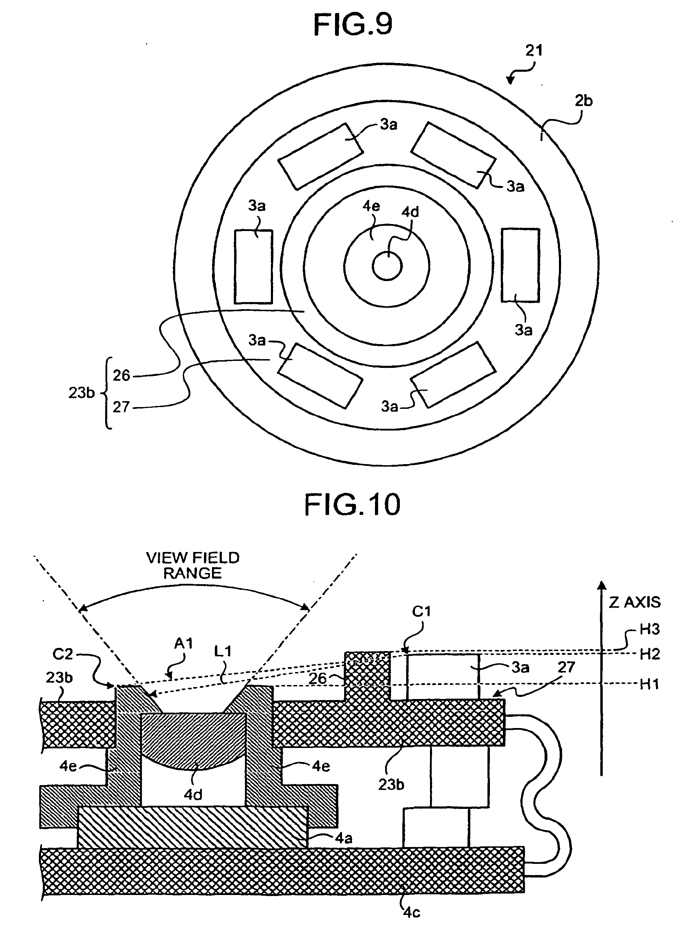

Next, the shield unit 26 formed integrally with the illumination board 23 is explained in detail. FIG. 9 is a schematic diagram illustrating the front surface of the capsule endoscope 21b as viewed from the direction D1 in FIG. 8. FIG. 10 is a schematic side sectional view showing an example of the shield unit 26 formed integrally with the illumination board 23b.

As shown in FIG. 9, the upper end portion of the lens frame 4e holding the lens 4d is exposed to the front surface of the illumination board 23b from the through hole at the central part of the illumination board 23b. Also, six light-emitting units 3a, for example, are mounted at rotationally symmetric positions, respectively, around the optical axis of the lens 4d in the cavity portion 27 of the illumination board 23b. On the front surface of this illumination board 23b, the annular shield unit 26, for example, is formed integrally with the illumination board 23b between the six light-emitting units 3a and the lens frame 4e. In this case, the annular shield unit 26 is formed in such a manner as to surround the upper end portion of the lens frame 4e.

The number of the light-emitting units 3a mounted in the cavity portion 27 is not specifically limited to six but may be any number capable of securing a sufficient light amount to illuminate the view field of the solid image sensor 4a to pick up the images in the subject vividly.

Also, the upper end portion of the lens frame 4e inserted in the through hole of the illumination board 23b is arranged at a position lower than the upper surface of the light-emitting units 3a mounted in the cavity portion 27. Specifically, as shown in FIG. 10, the height H1 of the upper end portion of the lens frame 4e is lower than the height H2 of the upper surface of the light-emitting units 3a mounted in the cavity portion 27. The shield unit 26 formed between the light-emitting units 3a and the upper end portion of the lens frame 4e has a sufficient height H3 to cross the plane A1 in contact with the crest C1 of each light-emitting unit 3a and the crest C2 of the upper end portion of the lens frame 4e. In this case, the shield unit 26 having this height H3 is located outside of the view field range specified by the lens 4d not to interrupt the view field of the solid image sensor 4a.

This shield unit 26 like the plural shield units 8 or the endless shield unit 16 described above, can block all the light paths (for example, the light path L1 described above) directed from the plural light-emitting units 3a on the cavity portion 27 toward the upper end portion of the lens frame 4e. In this way, the lens frame 4e can be positively shielded from all the light rays propagating from the plural light-emitting units 3a toward the upper end portion of the lens frame 4e. As a result, the shield unit 26, like the plural shield units 8 or the endless shield unit 16 described above, can prevent the light reflection at the upper end portion of the lens frame 4e while at the same time preventing the generation of a flare which otherwise might be caused by the light reflection at the upper end portion of the lens frame 4e.

Also, this shield unit 26 is formed integrally with the illumination board 23b (i.e. constitutes a part of the illumination board 23b) having the light-emitting units 3a and the lens frame 4e. The capsule endoscope 21 having the illumination board 23b integrally formed with the shield unit 26, therefore, can exhibit the function of shielding the lens frame 4e in the absence of a new shield unit formed separately from the illumination board 23b. Specifically, the capsule endoscope 21 having this configuration, as compared with the conventional capsule endoscope lacking the shield function described above, can exhibit the function of shielding the lens frame 4e without increasing the component members.

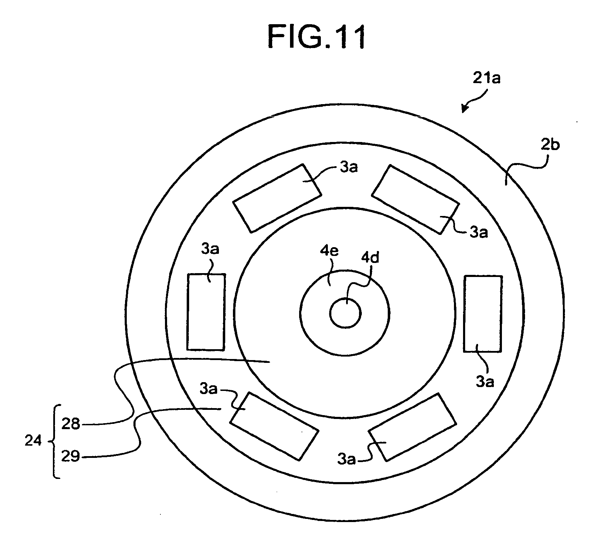

Next, a modification of the second embodiment of the invention is explained. FIG. 11 is a schematic front view showing an example of the configuration of the capsule endoscope according to a modification of the second embodiment of the invention. The shield unit for shielding the upper end portion of the lens frame 4e from the illumination light of the light-emitting units 3a is not necessarily formed as a protruded part of the illumination board 23b as described above, but a cavity portion (i.e. the portion having mounted the light-emitting units 3a therein) on the front surface of the illumination board may be formed at a lower level than the remaining part of the front surface and the low internal part of the cavity portion may be used as a shield unit.

Specifically, as shown in FIG. 11, the capsule endoscope 21a according to this modification of the second embodiment has an illumination board 24 in place of the illumination board 23b of the capsule endoscope 21 according to the second embodiment described above. This illumination board 24 is formed with a shield unit 28 at a high level outside of the upper end portion of the lens frame 4e, and the cavity portion 29 is formed at a low level outside of the shield unit 28. The other parts of the configuration are identical with the corresponding ones, respectively, of the second embodiment and designated by the same reference numerals, respectively.

The illumination board 24 is a disk-shaped rigid board formed with the circuit for realizing the function of the illumination system 23, and substantially similarly to in the capsule endoscope 1 according to the second embodiment described above, has plural (for example, six) light-emitting units 3a and the upper end portion of the lens frame 4e. Specifically, the illumination board 24 is formed with the annular shield unit 28, for example, along the outer periphery of the upper end portion of the lens frame 4e inserted in the through hole formed at the central part thereof, and a cavity portion 29 is formed along the outer periphery of the shield unit 28. In this case, the plural light-emitting units 3a are mounted on the cavity portion 29. Also, the shield unit 28 is located between the light-emitting units 3a mounted on the cavity portion 29 and the upper end portion of the lens frame 4e.

The number of the light-emitting units 3a mounted in the cavity portion 29 is not specifically limited to six but may be any number capable of securing a sufficient light amount to illuminate the view field of the solid image sensor 4a to pick up the images in the subject vividly.

The shield unit 28 is an endless (for example, annular) partial area formed integrally with the illumination board 24 at a position between the light-emitting units 3a and the upper end portion of the lens frame 4e and functions as a shield means for blocking the illumination light of the light-emitting units 3a directed toward the upper end portion of the lens frame 4e inserted in the through hole of the illumination board 24. In this case, the endless shield unit 28 is formed in such a manner as to surround the upper end portion of the lens frame 4e. This shield unit 28, like the endless shield unit 26 described above, positively blocks all the light rays propagating from the plural light-emitting units 3a toward the upper end portion of the lens frame 4e.

The cavity portion 29 is a part lower in level than the shield unit 28 on the front surface of the illumination board 24 and formed on the outer periphery of the shield unit 28. The plural light-emitting units 3a are mounted in the cavity portion 29 as described above. By forming the cavity portion 29 at low level in the neighborhood of the outer periphery of the front surface of the illumination board 24 in this way, the shield unit 28 higher in level than the cavity portion 29 is formed inside the cavity portion 29.

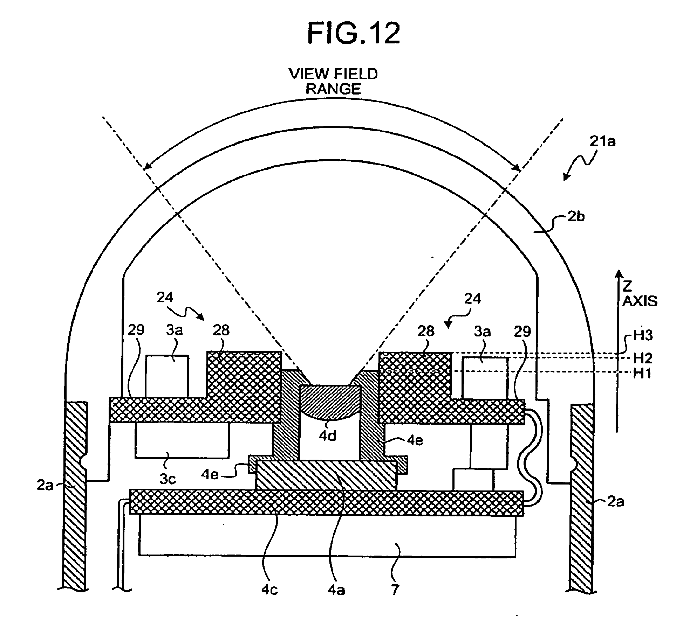

Next, the shield unit 28 formed integrally with the illumination board 24 is explained in detail. FIG. 12 is a schematic side sectional view showing an example of the shield unit 28 of the capsule endoscope 21a according to a modification of the second embodiment of the invention.

As shown in FIG. 12, the upper end portion of the lens frame 4e inserted in the through hole of the illumination board 24 is arranged at a lower position than the upper surface of the light-emitting units 3a mounted in the cavity portion 29 lower in level. In this case, the height H1 of the upper end portion of the lens frame 4e is lower than the height H2 of the upper surface of the light-emitting units 3a mounted in the cavity portion 29. Also, the shield units 28 formed on the inside of the low-level cavity portion 29 (i.e. a position between the light-emitting units 3a and the upper end portion of the lens frame 4e) are at a level higher than the cavity portion 29 on the illumination board 24 and have a sufficient height H3 to cross the plane A1 (see FIG. 10) in contact with the crest C1 of each light-emitting unit 3a and the crest C2 of the upper end portion of the lens frame 4e. In this case, the shield unit 28 having this height H3 is located outside the view field range defined by the lens 4d not to interrupt the view field of the solid image sensor 4a.

The shield unit 28, like the plural shield units 8 or the endless shield unit 26, can block all the light paths (including, for example, the light path L1 described above) directed from the plural light-emitting units 3a on the cavity portion 29 toward the upper end portion of the lens frame 4e, so that the lens frame 4e can be positively shielded from all the light rays propagating from the plural light-emitting units 3a toward the upper end portion of the lens frame 4e. As a result, the shield unit 26, like the plural shield units 8 or the endless shield unit 26 described above, can prevent the light reflection at the upper end portion of the lens frame 4e on the one hand and can suppress the generation of a flare which otherwise might be caused by the light reflection at the upper end portion of the lens frame 4e on the other hand. The capsule endoscope 21a having the illumination board 24 formed integrally with this shield unit 28 enjoys the same operation-effect as the capsule endoscope 21 according to the second embodiment described above.

According to the second embodiment and the modification thereof described above, the annular shield unit is formed integrally with the illumination board between the light-emitting units 3a and the upper end portion of the lens frame 4e. Nevertheless, the invention is not limited to this configuration, and the shield unit formed integrally with the illumination board may be replaced by an endless shield unit formed in a polygon, an ellipse, etc. in such a manner as to surround the lens frame 4e or, as indicated by the shield units 8 of the first embodiment, for example, the front surface of the illumination board may be partially protruded or raised to a high level in accordance with the plural light-emitting units 3a. The shield unit(s) formed integrally or partially on the illumination board in this way has the width W1 as described above.

As explained above, the second embodiment of the invention and the modification thereof are so configured that the circuit board (the illumination board described above) having the light-emitting units and the lens frame similar to those of the first embodiment has formed thereon the shield unit integrated with the particular circuit board at a position between the light-emitting units and the upper end portion of the lens frame, and at least that area of the upper end portion of the lens frame which otherwise would receive the illumination light from the light-emitting units (the upper end surface or the crest facing the light-emitting units, for example) is shielded by the shield unit from the illumination light of the light-emitting units. Without arranging a new shield unit as a separate member on the circuit board, therefore, the same shield function as the first embodiment described above can be exhibited. As a result, this embodiment can enjoy the operation-effect of the first embodiment described above while at the same time realizing the capsule endoscope that can be fabricated without increasing the number of members.

Also, since this shield unit is formed integrally with the circuit board, the labor of externally mounting a shield unit as a separate member between the light-emitting units and the upper end portion of the lens frame is saved, with the result that the production cost of the capsule endoscope having this shield unit can be reduced.

Third embodiment

Next, a third embodiment of the invention is explained. In the first and second embodiments and each modification described above, the shield unit(s) is interposed between the light-emitting units 3a and the upper end portion of the lens frame 4e on the front surface of the illumination board 3b. According to the third embodiment, in contrast, each shield unit for shielding the upper end portion of the lens frame 4e from the illumination light of the light-emitting units 3a is arranged directly on the corresponding one of the light-emitting units 3a.

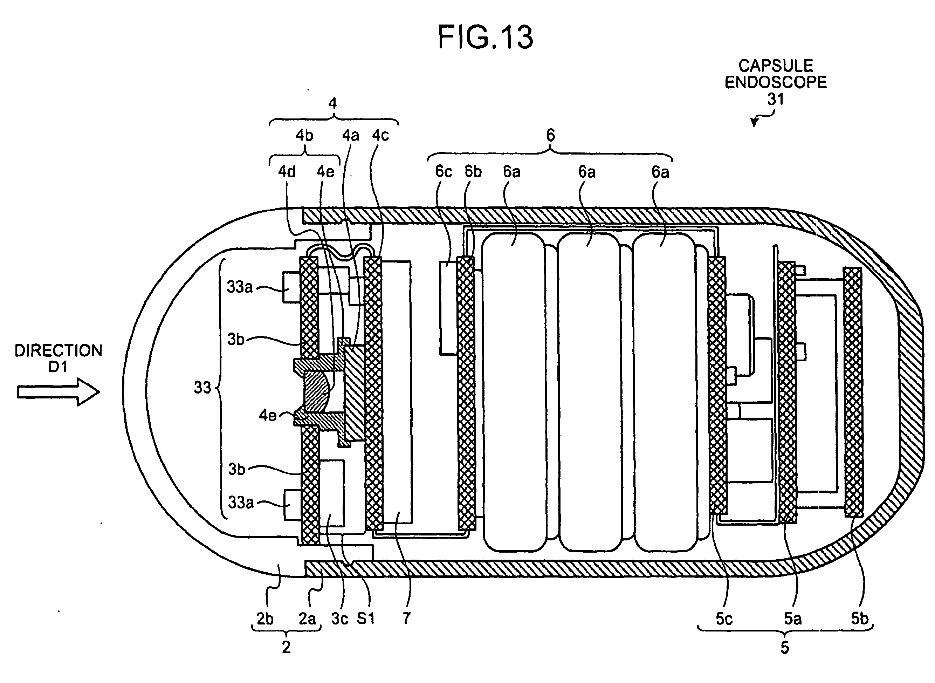

FIG. 13 is a schematic side sectional view showing an example of the configuration of the capsule endoscope according to the third embodiment of the invention. As shown in FIG. 13, the capsule endoscope 31 according to this embodiment has an illumination system 33 in place of the illumination system 3 of the capsule endoscope 1 according to the first embodiment described above. This illumination system 33 has light-emitting units 33a in place of the light-emitting units 3a of the illumination system 3 of the capsule endoscope 1 described above. The light-emitting units 33a each have the structure in which each shield unit is arranged directly on the side surfaces of the corresponding one of the light-emitting units 3a. Also, the illumination board 3b of the illumination system 33 lacks the shield units illustrated in the first and second embodiments and each modification described above. The other parts of the configuration are identical with the corresponding ones, respectively, of the first embodiment and designated by the same reference numerals, respectively.

The light-emitting units 33a have the structure in which each shield unit is arranged directly on the corresponding one of the light-emitting units 3a for emitting the illumination light for illuminating the view field of the solid image sensor 4a. Specifically, the light-emitting units 33a each have a frame-like shield unit directly arranged on the side surfaces of the corresponding one of the light-emitting units 3a, and like in the first embodiment described above, are mounted in the neighborhood of the outer periphery of the front surface of the illumination board 3b. In this case, the light-emitting units 33a illuminate the view field of the solid image sensor 4a while at the same time shielding the lens frame 4e from the illumination light.

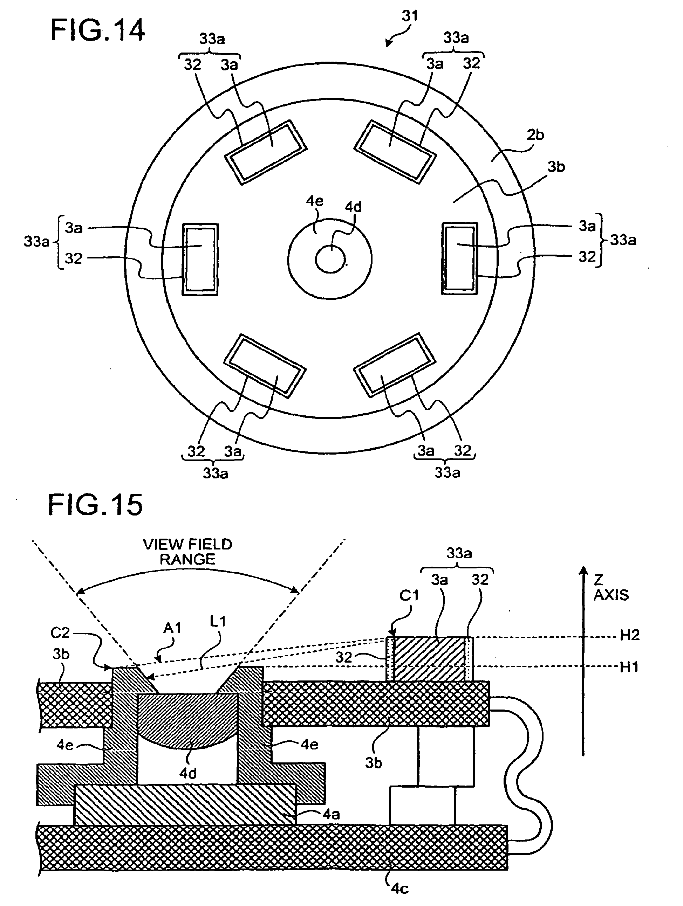

Next, the shield units of the capsule endoscope 31 according to the third embodiment are explained in detail. FIG. 14 is a schematic diagram illustrating the front surface of the capsule endoscope 31 as viewed from the direction D1 shown in FIG. 13. FIG. 15 is a schematic side sectional view showing an example of the shield unit arranged directly on the side surfaces of the corresponding light-emitting unit.

As shown in FIG. 14, the upper end portion of the lens frame 4e holding the lens 4d is exposed to the front surface of the illumination board 3b from the through hole at the central part of the illumination board 3b. Also, six light-emitting units 33a, for example, are mounted at rotationally symmetric positions, respectively, around the optical axis of the lens 4d in the neighborhood of the outer periphery of the illumination board 3b. The light-emitting units 33a each have the light-emitting unit 3a described above and the shield unit 32 arranged directly on the side surfaces of the light-emitting unit 3a. The shield units 32 are each a frame-like member, for example, covering the side surfaces of the light-emitting unit 3a and function as a light shield means for shielding the upper end portion of the lens frame 4e from the illumination light of the light-emitting units 3a.

The number of the light-emitting units 33a mounted on the illumination board 3b is not specifically limited to six but may be any number which can secure a sufficient light amount to illuminate the view field of the solid image sensor 4a to pick up images in the subject vividly.

Also, as shown in FIG. 15, the height H1 of the upper end portion of the lens frame 4e is lower than the height H2 of the upper surface of each light-emitting unit 3a on which the shield unit 32 is arranged directly. Each of the shield units 32 arranged in such a manner as to directly cover the side surfaces of the corresponding one of the light-emitting units 3a has a sufficient height to cross the plane A1 in contact with the crest C1 of the light-emitting unit 3a and the crest C2 of the upper end portion of the lens frame 4e. In this case, the height of the shield unit 32 is equal to or larger than the height H2 of the light-emitting unit 3a.

Each shield unit 32 arranged directly on each of the plural light-emitting units 3a can block each of the light paths (for example, the light path L1 described above) directed from the light-emitting units 3a toward the upper end portion of the lens frame 4e, thereby making it possible to shield the lens frame 4e positively from all the light rays propagating from the plural light-emitting units 3a toward the upper end portion of the lens frame 4e. As a result, the shield units 32 can prevent the light reflection at the upper end portion of the lens frame 4e on the one hand and can prevent the generation of a flare which otherwise might be caused by the light reflection at the upper end portion of the lens frame 4e at the same time.

Also, these shield units 32, as described above, are each arranged directly on the side surfaces of the corresponding light-emitting unit 3a. The capsule endoscope 31 having the light-emitting units 3a with the shield units 26 arranged in advance on the side surfaces thereof (i.e. the light-emitting units 33a) can have the function to shield the lens frame 4e as described above without providing a shield unit anew on the illumination board 3b. Specifically, the capsule endoscope 31 having this configuration, as compared with the conventional capsule endoscope lacking the shield function described above, can exhibit the function of shielding the lens frame 4e without increasing the number of members.

According to the third embodiment described above, the frame-like shield unit 32 is arranged directly on the side surfaces of each light-emitting unit 3a. The invention, however, is not limited to this configuration, and a colored (for example, black) resin or metal shield film may be formed directly on the side surfaces of the light-emitting units 3a. In this case, the shield film may be formed in such a manner as to cover at last the side surface of each light-emitting unit 3a facing the upper end portion of the lens frame 4e. Also, this shield film may be formed by painting black or coating a black adhesive or the like on the side surfaces of each light-emitting unit 3a.

As explained above, the third embodiment of the invention, like the first embodiment described above, is so configured that the shield unit is arranged directly on the side surfaces of each light-emitting unit, and the light-emitting units and the upper end portion of the lens frame are arranged on the circuit board (the illumination board described above) in such a manner that the shield units on the side surface of the light-emitting units face the upper end portion of the lens frame; so that the shield units on the side surfaces of the light-emitting units shield the upper end portion of the lens frame from the illumination light of the light-emitting units. Without providing a new shield unit on the circuit board, therefore, the shield function similar to that of the first embodiment described above can be secured. As a result, a capsule endoscope can be realized which can enjoy the operation-effect of the first embodiment described above and can be fabricated without increasing the number of members.

Also, in view of the fact that the shield units are arranged directly on the side surfaces of the light-emitting units and the resulting light-emitting units with the shield units arranged thereon in advance are mounted on the circuit board, it is possible to save the labor of externally mounting a separate shield unit between the light-emitting units and the upper end portion of the lens frame on the circuit board, with the result that the production cost of the capsule endoscope having these shield units can be reduced.

Fourth embodiment

Next, a fourth embodiment of the invention is explained. Unlike the first to third embodiments and each modification thereof described above having the shield units for shielding the upper end portion of the lens frame 4e from the illumination light of the light-emitting units 3a, the fourth embodiment is such that the shape of the upper end portion of the lens frame exposed to the front surface of the illumination board 3b is changed so that the light reflected at the upper end portion of the lens frame proceeds toward a position outside the view field of the solid image sensor 4a in the optical dome 2b.

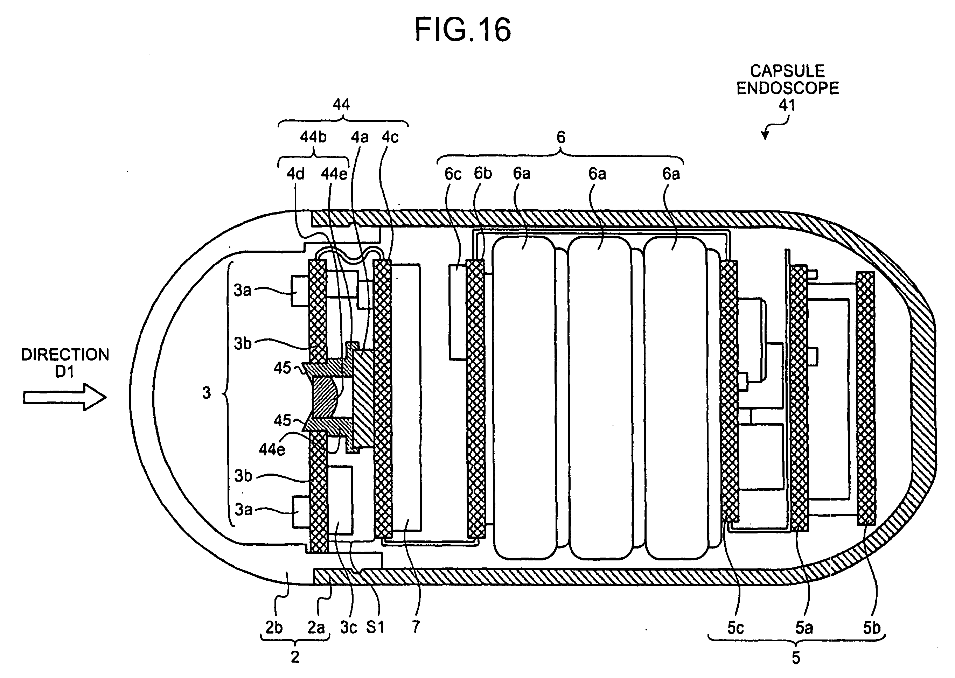

FIG. 16 is a schematic side sectional view showing an example of the configuration of the capsule endoscope according to a fourth embodiment of the invention. As shown in FIG. 16, the capsule endoscope 41 according to the fourth embodiment has an imaging system 44 in place of the imaging system 4 of the capsule endoscope 1 according to the first embodiment described above. This imaging system 44 has an optical system 44b in place of the optical system 4b of the imaging system 4 of the capsule endoscope 1 described above. This optical system 44b has a lens frame 44e in place of the lens frame 4e of the optical system 4b of the capsule endoscope 1 described above. The other parts of the configuration are identical with the corresponding ones, respectively, of the first embodiment and designated by the same reference numerals, respectively.

The lens frame 44e is different from the lens frame 4e of the capsule endoscope 1 according to the first embodiment in the shape of the upper end portion exposed to the front surface of the illumination board 3b. Specifically, the lens frame 44e, like the lens frame 4e described above, is inserted in the through hole of the illumination board 3b in such a manner as to hold the lens 4d at a position lower than the upper surface of the light-emitting units 3a. In this case, the upper end surface of the lens frame 44e is arranged at a position lower than the upper surface of the light-emitting units 3a. The upper end surface 45 of the lens frame 44e is tilted with respect to the upper end surface of the lens 4d less than the upper end surface of the lens frame 4e described above, so that the illumination light from the light-emitting units 3a is reflected at a position outside the view field of the solid image sensor 4a.

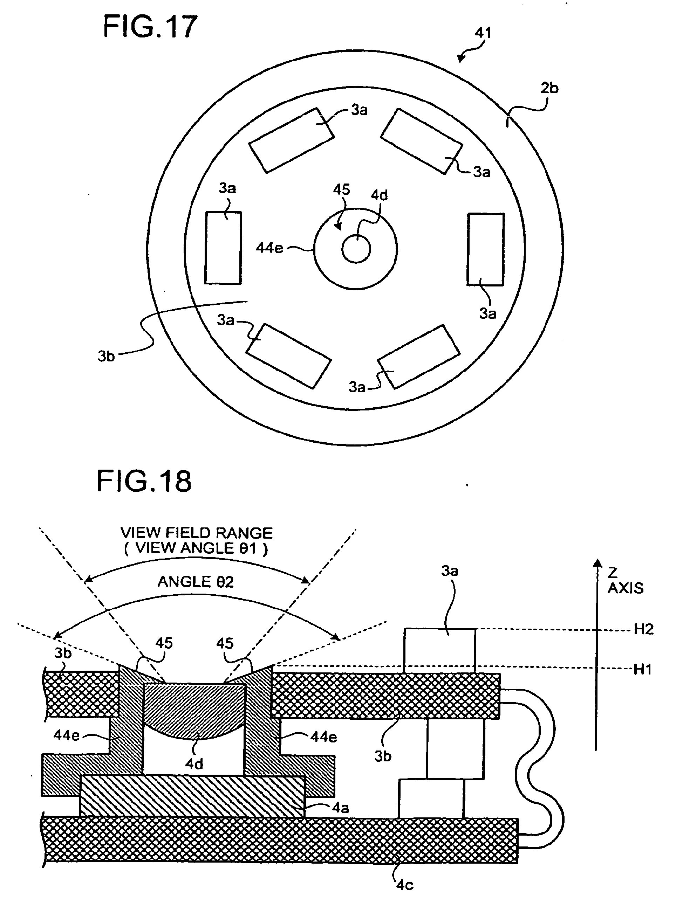

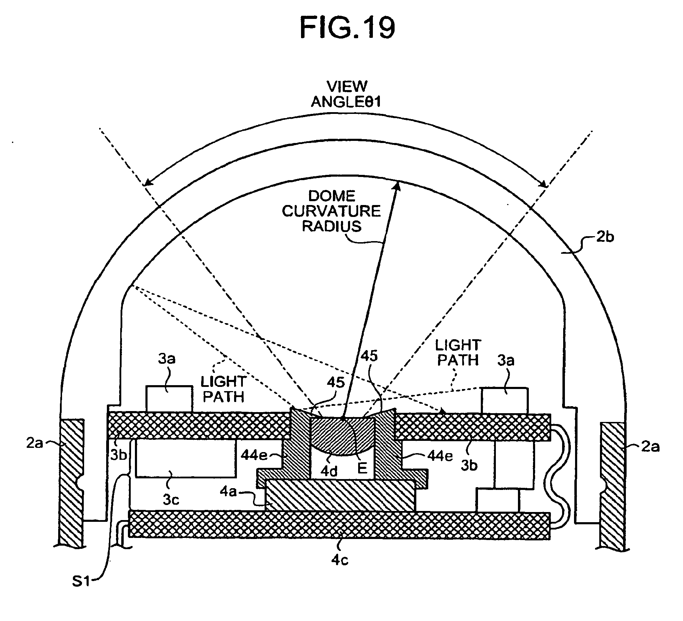

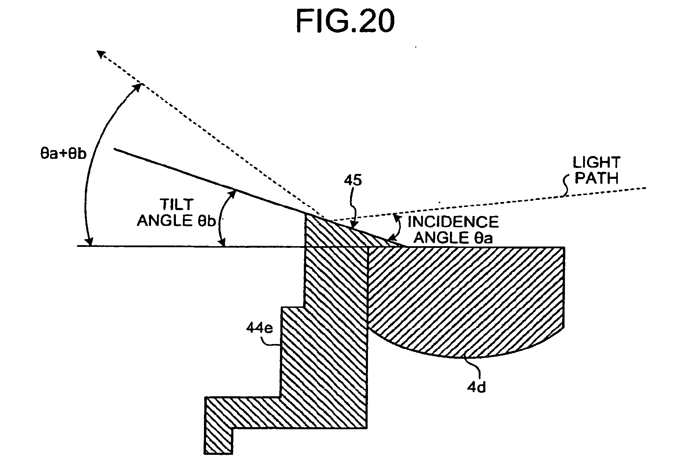

Next, the upper end portion of the lens frame 44e inserted in the through hole of the illumination board 3b is explained in detail. FIG. 17 is a schematic diagram illustrating the front surface of the capsule endoscope 41 as viewed from the direction D1 shown in FIG. 16. FIG. 18 is a schematic side sectional view showing an example of the upper end portion of the lens frame 44e reflecting the illumination light from the light-emitting units 3a at a position outside the view field of solid image sensor 4a. FIG. 19 is a schematic sectional view explaining the structure of the upper end portion of the lens frame 44e in more detail. FIG. 20 is an enlarged schematic diagram showing the lens frame 44e in FIG. 19. An example of the light paths of the illumination light, before reaching a position outside the view field of solid image sensor 4a sequentially through the lens frame 44e and the optical dome 2b, is shown by dotted arrows in FIGS. 19 and 20.

As shown in FIG. 17, the upper end portion of the lens frame 44e holding the lens 4d is inserted in the through hole at the central part of the illumination board 3b. In this case, the upper end surface 45 of the lens frame 44e is exposed to the front surface of the illumination board 3b. As described above, the plural light-emitting units 3a are mounted on the front surface of the illumination board 3b.

Also, the upper end portion of the lens frame 44e inserted in the through hole of illumination board 3b is arranged at a position lower than the upper surface of the light-emitting units 3a. Specifically, as shown in FIG. 18, the height H1 of the upper end portion of the lens frame 44e is lower than the height H2 of the upper surface of the light-emitting units 3a mounted on the front surface of the illumination board 3b. The upper end surface 45 of the lens frame 44e is formed in the shape (for example, a slope) tilted less than the upper end surface of the lens frame 4e described above with respect to the upper surface of the lens 4d. In this case, the angle θ2 formed by the upper end surface 45 is larger than the angle formed by the upper end surface of the lens frame 4e, and realizes the upper end surface 45 which reflects the illumination light of the light-emitting units 3a at a position outside the view field of the solid image sensor 4a. This angle θ2, larger than the angle specifying the range of the view field of solid image sensor 4a (i.e. the view angle θ1 of solid image sensor 4a), is set based on the relative positions of the upper end portion of the lens frame 44e and the optical dome 2b, the angle of the illumination light incident to the upper end portion of the lens frame 44e and the view angle θ1.

More specifically, as shown in FIGS. 19 and 20, the tilt angle θb of the upper end surface 45 with respect to the lens 4d is set in such a manner as to satisfy Equation (1) below including the angle θa of the illumination light incident to the upper end surface 45 of the lens frame 4e and the view angle θ1 of the solid image sensor 4a. The angle θ2 formed by the upper end surface 45 is specified by the tilt angle θb of the upper end surface 45 and calculated from Equation (2) below.

$$(180° - \text{view angle } θ1)/2 > \text{incidence angle } θa + \text{tilt angle } θb \tag{1}$$

$$\text{Angle } θ2 = 180° - 2 × \text{tilt angle } θb \tag{2}$$

Also, the lens 4d held by the lens frame 44e, as shown in FIG. 19, is fixedly arranged in such a manner that the center of curvature of the dome shape forming the curvature radius of the optical dome 2b (the dome curvature radius) coincident with the entrance pupil center E of the lens 4d.

By satisfying the angular conditions of the tilt angle θb (or the angle θ2) of the upper end surface 45 as shown above, the flare is prevented from being generated by the light reflection at the upper end portion of the lens frame 44e even in the case where the illumination light from the light-emitting units 3a reaches the upper end portion of the lens frame 44e. Also, by further satisfying the layout conditions of the lens 4d described above, the flare which otherwise might be generated by the light reflection at the upper end portion of the lens frame 44e can be more positively suppressed.

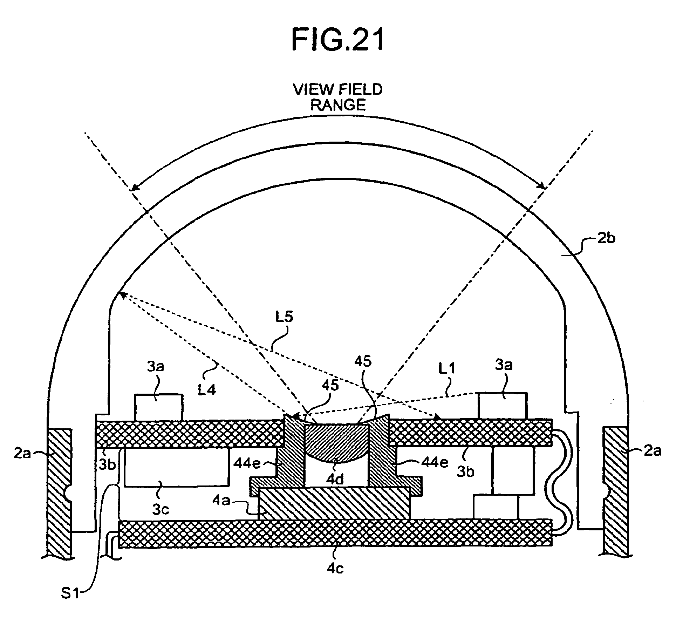

Next, an explanation is given about the operation of the upper end surface 45 of the lens frame 44e to reflect the illumination light from the light-emitting units 3a at a position outside of the view field of solid image sensor 4a. FIG. 21 is a schematic side sectional view explaining the reflect operation at the upper end portion of the lens frame 44e for reflecting the illumination light from the light-emitting units 3a at a position outside of the view field of the solid image sensor 4a.

As shown in FIG. 21, the side surface of the cylindrical portion of the lens frame 44e as close to the upper end portion as possible is fitted in the through hole of the illumination board 3b to secure the internal space S1 for arranging the function part 3c described above without increasing the size (casing size) of the capsule endoscope. The lens frame 44e, in which the side surface of the cylindrical portion close to the upper end portion is inserted in the through hole of the illumination board 3b, has the upper end surface 45 thereof located at a lower position than the upper surface of the light-emitting units 3a and holds the lens 4d at a position lower than the upper surface of the light-emitting units 3a.

In the case where the light-emitting units 3a and the lens frame 44e are arranged in these relative positions, part of the illumination light emitted from the light-emitting units 3a reaches the upper end surface 45 of the lens frame 44e by propagating through, for example, the light path L1 while at the same time being reflected by the upper end surface 45. This upper end surface 45, forming the angle θ2 described above, is a gentle slope at a small angle with respect to the upper surface of the lens 4d, for example. This upper end surface 45, as shown in FIG. 21, reflects the illumination light from the light-emitting units 3a at a position outside of the view field of solid image sensor 4a. Specifically, the light reflected on the upper end surface 45 propagates through the light path L4, for example, and reaches a position outside of the view field range of the optical dome 2b (i.e. outside of the view field of solid image sensor 4a), while at the same time being reflected by the optical dome 2b at the position outside the view field range. After that, the light reflected at the position outside the view field range of the optical dome 2b propagates through the light path L5, for example, and reaches the outside of the lens frame 44e (for example, the front surface of illumination board 3b).

In this way, the upper end surface 45 of the lens frame 44e, by reflecting the light from the light-emitting units 3a at a position outside of the view field of the solid image sensor 4a, can prevent the unrequired light from entering the lens 4d from the light-emitting units 3a. The capsule endoscope 41 having the lens frame 44e forming this upper end surface 45 can prevent the generation of a flare which otherwise might be caused by the light reflection at the upper end portion of the lens frame 44e even in the case where the illumination light reaches the upper end portion of the lens frame 44e from the light-emitting units 3a.

According to the fourth embodiment of the invention, the upper end surface 45 of the lens frame 44e forms a slope tilted with respect to the upper surface of the lens 4d. Nevertheless, the invention is not limited to this configuration, but the upper end surface of the lens frame 44e may be substantially parallel to the upper surface of the lens 4d (i.e. not tilted). In such a case, the angle 02 formed by the upper end surface of the lens frame 44e is about 180 degrees.

As described above, according to the fourth embodiment of the invention, the upper end surface of the lens frame exposed to the front surface of the circuit board (the illumination board described above) having mounted thereon the light-emitting units for illuminating the view field of the solid image sensor is formed at such an angle as to reflect the illumination light from the light-emitting units at a position outside of the view field in the optical dome. As a result, the unrequired light from the light-emitting units is prevented from entering the lens held by the lens frame. As a result, a capsule endoscope can be realized wherein even in the case where the illumination light reaches the upper end portion of the lens frame from the light-emitting units, the flare which otherwise might be generated by the light reflection at the upper end portion of the lens frame can be suppressed.

Also, the labor can be saved which otherwise would be required to arrange the shield unit for shielding the upper end portion of the lens frame from the illumination light of the light-emitting units, on the circuit board or the side surfaces of the light-emitting units. As a result, the capsule endoscope can be fabricated without increasing the number of members and the production cost of the capsule endoscope can be reduced.

INDUSTRIAL APPLICABILITY

As described above, the capsule endoscope according to this invention is useful to acquire the images in the organs of the subject, or especially suitable to prevent the generation of a flare which otherwise might be caused by the fact that the illumination light illuminating the interior of the organs is reflected on the lens frame of the imaging means.

**Claims**

1. A capsule endoscope (1; 1a; 1b; 1c; 21; 21a; 31) having a solid image sensor (4a) introduced into a subject for picking up an image in the subject, comprising:

   a light-emitting unit (3a; 33a) for emitting illumination tight for !!!uminating a view field of the solid image sensor (4a);
   a lens frame (4e) for holding a lens to focus an image in the subject on a light-receiving surface of the solid image sensor (4a), the lens frame (4e) having an upper end portion located at a position lower than an upper surface of the light-emitting unit (3a; 33a);
   a shield unit (26) for shielding, from the illumination light, at least a partial area of the upper end portion of the lens frame (4e) which receives the illumination light; and
   a circuit board (23b) having a through hole into which the lens frame (4e) is inserted, the light-emitting unit (3a) being mounted on a surface of the circuit board (23b), **characterized in that**
   the shield unit (26) is disposed on the circuit board (23b) and between the upper end portion of the lens frame (4e) and the light-emitting unit (3a), and
   the shield unit (26) is formed integrally on the circuit board (23b).

2. The capsule endoscope (1; 1a) according to claim 1, wherein the shield unit (8) has such a height that the shield unit (8) crosses a plane (A1) which comes in contact with a crest of the upper surface of the light-emitting unit (3a) close to the lens frame (4e) and a crest of the upper end portion of the lens frame (4e).

3. The capsule endoscope (1; 1a) according to claim 2, wherein the crest of the upper end portion of the lens frame (4e) in contact with the plane (A1) is located at the farthest end from the light-emitting unit (3a).

4. The capsule endoscope (1c) according to claim 1, wherein the shield unit (17) is formed by curing a paste-like resin coated on the circuit board (3b).

5. The capsule endoscope (1a) according to claim 1, wherein the shield unit (15) is a chip part.

6. The capsule endoscope (1b; 21; 21a) according to claim 1, wherein the shield unit (16; 26; 28) is formed to be such an endless shape as to surround the upper end portion of the lens frame (4e).

7. The capsule endoscope (31) according to claim 1, wherein the shield unit (32) is disposed directly on a side surface of the light-emitting unit (33a) facing at least the upper end portion of the lens frame (4e).

8. The capsule endoscope (31) according to claim 7, wherein the shield unit (32) is a frame member surrounding the side surface of the light-emitting unit (3a).

**Patentansprüche**

1. Gekapseltes Endoskop (1; 1a; 1b; 1c; 21; 21a; 31) mit einem in einen Patienten eingeführten Festkörper-Bildsensor (4a), um in dem Patienten ein Bild aufzunehmen, mit:

    einer lichtemittierenden Einheit (3a; 33a) zum Emittieren von Beleuchtungslicht zum Beleuchten eines Sichtfeldes des Festkörper-Bildsensors (4a);
    einem Linsenrahmen (4e) zum Halten einer Linse, um ein Bild in dem Patienten auf einer Lichtempfangsfläche des Festkörper-Bildsensors (4a) zu fokussieren, wobei der Linsenrahmen (4e) einen oberen Endabschnitt hat, der sich an einer niedrigeren Position befindet als eine obere Oberfläche der lichtemittierenden Einheit (3a; 33a);
    einer Abschirmeinheit (26) zum Abschirmen zumindest eines Teilbereichs des oberen Endabschnitts des Linsenrahmens (4e), der das Beleuchtungslicht empfängt, von dem Beleuchtungslicht; und
    einer Schaltungsplatte (23b) mit einem Durchgangsloch, in das der Linsenrahmen (4e) eingesetzt ist, wobei die lichtemittierende Einheit (3a) auf einer Oberfläche der Schaltungsplatte (23b) angebracht ist,
    **dadurch gekennzeichnet, dass**
    die Abschirmeinheit (26) auf der Schaltungsplatte (23b) und zwischen dem oberen Endabschnitt des Linsenrahmens (4e) und der lichtemittierenden Einheit (3a) angeordnet ist, und
    die Abschirmeinheit (26) integral auf der Schaltungsplatte (23b) ausgebildet ist.

2. Gekapseltes Endoskop (1; 1a) nach Anspruch 1, wobei die Abschirmeinheit (8) eine solche Höhe hat, dass die Abschirmeinheit (8) eine Ebene (A1) kreuzt, die mit einem Scheitel der oberen Oberfläche der lichtemittierenden Einheit (3a) nahe dem Linsenrahmen (4e) und einem Scheitel des oberen Endabschnitts des Linsenrahmens (4e) in Kontakt kommt.

3. Gekapseltes Endoskop (1; 1a) nach Anspruch 2, wobei der in Kontakt mit der Ebene (A1) stehende Scheitel des oberen Endabschnitts des Linsenrahmens (4e) an dem von der lichtemittierenden Einheit (3a) am weitesten entfernten Ende gelegen ist.

4. Gekapseltes Endoskop (1c) nach Anspruch 1, wobei die Abschirmeinheit (17) durch Aushärten eines auf die Schaltungsplatte (3b) durch Beschichtung aufgebrachten pastenartigen Harzes ausgebildet ist.

5. Gekapseltes Endoskop (1a) nach Anspruch 1, wobei die Abschirmeinheit (15) ein Chip-Teil ist.

6. Gekapseltes Endoskop (1b; 21; 21a) nach Anspruch 1, wobei die Abschirmeinheit (16; 26; 28) derart ausgebildet ist, dass sie in einer Endlosform den oberen Endabschnitt des Linsenrahmens (4e) umgibt.

7. Gekapseltes Endoskop (31) nach Anspruch 1, wobei die Abschirmeinheit (32) direkt an einer Seitenfläche der lichtemittierenden Einheit (33a) angeordnet ist, welche zumindest dem oberen Endabschnitt des Linsenrahmens (4e) zugewandt ist.

8. Gekapseltes Endoskop (31) nach Anspruch 7, wobei die Abschirmeinheit (32) ein Rahmenelement ist, das die Seitenfläche der lichtemittierenden Einheit (3a) umgibt.

**Revendications**

1. Endoscope encapsulé (1 ; 1a ; 1b ; 1c ; 21 ; 21a ; 31) comportant un capteur d'image solide (4a) introduit dans un sujet pour capturer une image dans le sujet, comprenant :

une unité d'émission de lumière (3a ; 33a) destinée à émettre une lumière d'éclairage pour éclairer un champ de vision du capteur d'image solide (4a) ;
une monture d'objectif (4e) destinée à maintenir un objectif pour focaliser une image dans le sujet sur une surface de réception de lumière du capteur d'image solide (4a), la monture d'objectif (4e) comportant une partie d'extrémité supérieure placée au niveau d'une position inférieure à une surface supérieure de l'unité d'émission de lumière (3a ; 33a) ;
une unité de protection (26) destinée à protéger de la lumière d'éclairage au moins une zone partielle de la partie d'extrémité supérieure de la monture d'objectif (4e) qui reçoit la lumière d'éclairage ; et
une carte de circuit imprimé (23b) comportant un trou traversant dans lequel la monture d'objectif (4e) est insérée, l'unité d'émission de lumière (3a) étant montée sur une surface de la carte de circuit imprimé (23b),
**caractérisé en ce que**
l'unité de protection (26) est disposée sur la carte de circuit imprimé (23b) et entre ia partie d'extrémité supérieure de la monture d'objectif (4e) et l'unité d'émission de lumière (3a), et
l'unité de protection (26) est formée solidairement sur la carte de circuit imprimé (23b).

2. Endoscope encapsulé (1 ; 1a) selon la revendication 1, dans lequel l'unité de protection (8) présente une hauteur telle que l'unité de protection (8) traverse un plan (A1) qui vient en contact avec un sommet de la surface supérieure de l'unité d'émission de lumière (3a) proche de la monture d'objectif (4e) et un sommet de la partie d'extrémité supérieure de la monture d'objectif (4e).

3. Endoscope encapsulé (1 ; 1a) selon la revendication 2, dans lequel le sommet de la partie d'extrémité supérieure de la monture d'objectif (4e) en contact avec le plan (A1) est placé au niveau de l'extrémité la plus éloignée de l'unité d'émission de lumière (3a).

4. Endoscope encapsulé (1c) selon la revendication 1, dans lequel l'unité de protection (17) est formée en durcissant une résine de type pâte revêtue sur la carte de circuit imprimé (3b).

5. Endoscope encapsulé (1a) selon la revendication 1, dans lequel l'unité de protection (15) est une partie de puce.

6. Endoscope encapsulé (1b ; 21 ; 21a) selon la revendication 1, dans lequel l'unité de protection (16 ; 26 ; 28) est formée pour présenter une forme sans fin de façon à entourer la partie d'extrémité supérieure de la monture d'objectif (4e).

7. Endoscope encapsulé (31) selon la revendication 1, dans lequel l'unité de protection (32) est disposée directement sur une surface latérale de l'unité d'émission de lumière (33a) en regard d'au moins la partie d'extrémité supérieure de la monture d'objectif (4e).

8. Endoscope encapsulé (31) selon la revendication 7, dans lequel l'unité de protection (32) est un élément de monture entourant la surface latérale de l'unité d'émission de lumière (3a).

FIG.1

# FIG.2

# FIG.3

# FIG.4

VIEW FIELD
RANGE

# FIG.5

# FIG.6

# FIG.7

# FIG.8

EP 2 011 429 B1

CAPSULE
ENDOSCOPE
21

# FIG.9

# FIG.10

# FIG.11

# FIG.12

VIEW FIELD
RANGE

FIG.13

## FIG.14

## FIG.15

FIG.16

## FIG.17

## FIG.18

# FIG.19

VIEW
ANGLEθ1

DOME
CURVATURE
RADIUS

2b

3a

LIGHT
PATH

LIGHT
PATH

3a

45

45

2a

3b

44e

44e

3b

2a

3c

4d

E

4a

4c

S1

# FIG.20

# FIG.21

VIEW FIELD
RANGE

**EP 2 011 429 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 00076391 A
- JP 2006068488 A
- JP 2003275171 A